# EUROPEAN PATENT APPLICATION

(11) **EP 2 717 345 A1**
(43) Date of publication of application: **09.04.2014**
(21) Application number: 12792862.0
(22) Date of filing: 16.05.2012
(51) Int. Cl.: H01L 51/40, C07K 14/80, H01L 21/331, H01L 29/73, H01L 29/74, H01L 31/10, H01L 51/05, H01L 51/30, H01L 51/42

(54) **METHOD FOR PRODUCING PROTEIN SEMICONDUCTOR**

(30) Priority: 27.05.2011 JP 2011119329
(71) Applicant: Sony Corporation, Tokyo 108-0075 (JP)
(72) Inventor: TOKITA, Yuichi, Tokyo 108-0075 (JP); GOTO, Yoshio, Tokyo 108-0075 (JP); LUO, Wei, Tokyo 108-0075 (JP); NAKAMARU, Satoshi, Tokyo 108-0075 (JP); YAMADA, Seiji, Tokyo 108-0075 (JP)
(74) Representative: Müller - Hoffmann & Partner
(86) International application number: PCT/JP2012/003200
(87) International publication number: WO 2012/164849

(57) **Abstract**

[Object] To provide a method of controlling a conductivity type of a protein semiconductor, which is capable of easily controlling a conductivity type of a protein semiconductor, a method of manufacturing a protein semiconductor using this, and a method of manufacturing a pn junction.

[Solving Means] A conductivity type of a protein semiconductor is controlled by controlling total amount of charge in amino acid residues, a p-type protein semiconductor or an n-type protein semiconductor is manufactured, and a pn junction is manufactured using the p-type protein semiconductor and the n-type protein semiconductor. The total amount of charge in amino acid residues is controlled by substituting one or more of an acidic amino acid residue, a basic amino acid residue, and a neutral amino acid residue, which are contained in protein, with an amino acid residue having different properties, chemically modifying one or more of an acidic amino acid residue, a basic amino acid residue, and a neutral amino acid residue, which are contained in the protein, or controlling polarity of a medium surrounding the protein.

## Description

### Technical Field

The present disclosure relates to a method of manufacturing a protein semiconductor, a protein semiconductor, a method of manufacturing a pn junction, a pn junction, a method of manufacturing a semiconductor apparatus, a semiconductor apparatus, an electronic apparatus, and a method of controlling a conductivity type of a protein semiconductor.

### Background Art

Protein is expected to be used as a next-generation function element or the material thereof instead of an existing semiconductor element using a semiconductor such as silicon. The minimum size of the existing semiconductor element is several ten nm. On the other hand, the protein fulfills an advanced and complicated function even with a very small size of 2 to 10 nm.

The protein is well known to have the properties of a semiconductor (see, for example, Non-Patent Document 1). However, it is considered that the protein has the properties as long as the protein itself has a band gap of 2 to 3 electron volt (eV). On the other hand, in order to manufacture a semiconductor element using a protein semiconductor, it is necessary to control a conductivity type of the protein semiconductor, i.e., to be able to control the protein semiconductor to be p-type or n-type.

### Citation List

### Patent Document

Patent Document 1: Japanese Patent Application Laid-open No. 2007-220445
Patent Document 2: Japanese Patent Application Laid-open No. 2009-21501

### Non-Patent Document

Non-Patent Document 1: D.D.Eley, R.B.Leslie: "Electronic Aspects of Biochemistry",Academic Press, New York(1964)p.105
Non-Patent Document 2: Nikkila,H.,Gennis,R.B.,and Sliger,S.G.Eur.J.Biochem.202,309(1991)
Non-Patent Document 3: Mathews,F.S.,Bethge,P.H.,and Czerwinski,E.W.J.Biol.Chem.254,1699(1979)
Non-Patent Document 4: Hamachi,I.,Takashima,H.,Tsukiji,S.Shinkai,S.,Nagamune,T.andOis hi,S.Chem.Lett.1999,551(1999)
Non-Patent Document 5: Itagaki,E.,Palmer,G.and Hager,L.P.J.Biol.Chem.242,2272(1967)
Non-Patent Document 6: Tokita,Y.and 4 others,J.Am.Chem.Soc.130,5302(2008)

### Disclosure of the Invention

### Problem to be solved by the Invention

However, there have been no means for controlling a conductivity type of a protein semiconductor as far as the present inventors know.

In view of the above, a problem to be solved by the present disclosure is to provide a method of controlling a conductivity type of a protein semiconductor, which is capable of easily controlling a conductivity type of a protein semiconductor, a method of manufacturing a protein semiconductor, and a protein semiconductor.

Another problem to be solved by the present disclosure is to provide a method of manufacturing a pn junction with a protein semiconductor, a pn junction, a method of manufacturing a semiconductor apparatus using the pn junction, a semiconductor apparatus, and an electronic apparatus including the semiconductor apparatus.

The above and other problems will be clear from the description of the present specification with reference to the accompanying drawings.

### Means for solving the Problem

In order to solve the above-mentioned problems, the present disclosure provides
a method of controlling a conductivity type of a protein semiconductor, including controlling the conductivity type of the protein semiconductor by controlling total amount of charge in amino acid residues.

Here, the conductivity type of the protein semiconductor is p-type, n-type, or i-type.

Moreover, the present disclosure provides
a method of manufacturing a protein semiconductor, including controlling a conductivity type of the protein semiconductor by controlling total amount of charge in amino acid residues.

Moreover, the present disclosure provides
a protein semiconductor whose conductivity type is controlled by controlling total amount of charge in amino acid residues.

Moreover, the present disclosure provides
a method of manufacturing a pn junction, including manufacturing a p-type protein semiconductor and an n-type protein semiconductor by controlling total amount of charge in amino acid residues, and manufacturing a pn junction by joining the p-type protein semiconductor and the n-type protein semiconductor together.

Moreover, the present disclosure provides
a pn junction manufactured by manufacturing a p-type protein semiconductor and an n-type protein semiconductor by controlling total amount of charge in amino acid residues, and joining the p-type protein semiconductor and the n-type protein semiconductor together.

Moreover, the present disclosure provides
a method of manufacturing a semiconductor apparatus, including the steps of manufacturing a p-type protein semiconductor and an n-type protein semiconductor by controlling total amount of charge in amino acid residues, and manufacturing a pn junction by joining the p-type protein semiconductor and the n-type protein semiconductor together.

Moreover, the present disclosure provides
a semiconductor apparatus, including a pn junction manufactured by manufacturing a p-type protein semiconductor and an n-type protein semiconductor by controlling total amount of charge in amino acid residues, and joining the p-type protein semiconductor and the n-type protein semiconductor together.

Moreover, the present disclosure provides
an electronic apparatus, including a semiconductor apparatus including a pn junction manufactured by manufacturing a p-type protein semiconductor and an n-type protein semiconductor by controlling total amount of charge in amino acid residues, and joining the p-type protein semiconductor and the n-type protein semiconductor together.

In order to control the total amount of charge in amino acid residues in the protein, in more detail, in a polypeptide portion of the protein, for example, one or more of an acidic amino acid residue, a basic amino acid residue, and a neutral amino acid residue, which are contained in protein, is substituted with an amino acid residue having different properties. Alternatively, one or more of an acidic amino acid residue, a basic amino acid residue, and a neutral amino acid residue are added to the protein. Alternatively, one or more of an acidic amino acid residue, a basic amino acid residue, and a neutral amino acid residue, which are contained in the protein, are deleted. Alternatively, one or more of an acidic amino acid residue, a basic amino acid residue, and a neutral amino acid residue, which are contained in the protein, are chemically modified. Alternatively, polarity of a medium surrounding the protein is controlled. These methods may be combined as necessary. Moreover, in some cases, the total amount of charge in amino acid residues can be controlled by photodoping, i.e., applying light to the protein to generate an electron-hole pair.

In the present disclosure, the protein is favorably electron transfer protein. The electron transfer protein is generally electron transfer protein containing metal. The metal is, favorably, transition metal having an electron in an orbital having higher energy than d orbital. Examples of the electron transfer protein include, but not limited to, iron-sulfur proteins (e.g., rubredoxin, iron (ii) ferredoxin, iron (iii) ferredoxin, and iron (iv) ferredoxin), blue copper proteins (e.g., plastocyanin, azurin, pseudoazurin, plantacyanin, stellacyanin, and amicyanin), and cytochromes (e.g., cytochrome c, metal-substituted cytochrome c, metal-substituted cytochrome c₅₅₂ obtained by substituting iron being central metal of heme of cytochrome c₅₅₂ with another metal, modified zinc porphyrin cytochrome c₅₅₂, cytochrome b, cytochrome b₅, cytochrome c₁, cytochrome a, cytochrome a₃, cytochrome f, cytochrome b₆, cytochrome b₅₆₂, metal-substituted cytochrome b₅₆₂, and zinc chlorine cytochrome b₅₆₂). For example, derivatives of the electron transfer protein (obtained by chemically modifying amino acid residues in the skeleton) or variants thereof (obtained by substituting a part of amino acid residues in the skeleton with another amino acid residue) may be used. Metals in metal-substituted cytochrome c, metal-substituted cytochrome c₅₅₂, and metal-substituted cytochrome b₅₆₂ are selected as necessary. Examples of the metals include zinc (Zn), beryllium (Be), strontium (Sr), niobium (Nb), barium (Ba), lutetium(Lu), hafnium (Hf), tantalum (Ta), cadmium (Cd), antimony (Sb), thorium (Th), and lead (Pb).

The semiconductor apparatus may be basically anything as long as it uses a pn junction (including a pin junction in which an intrinsic (i-type) protein semiconductor is sandwiched between a p-type protein semiconductor and an n-type protein semiconductor). The semiconductor apparatus is, specifically, a light-receiving element, a light emission element, an electric field detection element, a carrier transit element (e.g., transistor), or the like. Here, the electric field detection element can be configured by using not only the pn junction but also the p-type protein semiconductor alone or n-type protein semiconductor alone.

In the present disclosure, the total amount of charge in amino acid residues in the protein used as a starting material is controlled by various methods, i.e., substituting one or more of an acidic amino acid residue, a basic amino acid residue, and a neutral amino acid residue, which are contained in the protein, with an amino acid residue having different properties, thereby controlling a conductivity type of the obtained protein semiconductor.

### Effect of the Invention

According to the present disclosure, it is possible to easily control a conductivity type of a protein semiconductor. It is also possible to easily manufacture a pn junction with a protein semiconductor using the control method, and to easily attain a novel semiconductor apparatus by using the pn junction. Then, it is possible to attain a sophisticated electronic apparatus by using the semiconductor apparatus.

### Brief Description of Drawings

[Fig. 1] A schematic diagram for explaining a method of controlling a conductivity type of a protein semiconductor according to a first embodiment.
[Fig. 2] A schematic diagram showing a structure of zinc-substituted cytochrome c and positions of basic amino acid residues.
[Fig. 3] A schematic diagram showing a structure of zinc-substituted cytochrome b₅₆₂ and positions of basic amino acid residues.
[Fig. 4] A schematic diagram showing a structure of zinc-substituted cytochrome c and positions of neutral amino acid residues.
[Fig. 5] A schematic diagram showing a structure of zinc-substituted cytochrome b₅₆₂ and positions of neutral amino acid residues.
[Fig. 6] A schematic diagram showing an absorption spectrum of purified cytochrome b₅₆₂.
[Fig. 7] A schematic diagram showing a structure of cytochrome b₅₆₂.
[Fig. 8] A schematic diagram schematically showing a state where cytochrome b₅₆₂ adsorbs to a gold electrode via a self-assembled monolayer.
[Fig. 9] A schematic diagram showing a cyclic voltammogram obtained by using a cytochrome b₅₆₂-immobilized gold drop electrode.
[Fig. 10] A schematic diagram showing an absorption spectrum of zinc-substituted cytochrome b₅₆₂.
[Fig. 11] A schematic diagram showing photocurrent real-time waveforms obtained by using a zinc-substituted cytochrome b₅₆₂-immobilized gold drop electrode.
[Fig. 12] A schematic diagram showing a photocurrent action spectrum obtained by using a zinc-substituted cytochrome b₅₆₂-immobilized gold drop electrode.
[Fig. 13] A schematic diagram showing a current-voltage curve obtained by using a zinc-substituted cytochrome b₅₆₂-immobilized gold drop electrode.
[Fig. 14] A diagram showing a pn junction according to a third embodiment and an energy band of the pn junction at the time of zero bias.
[Fig. 15] A diagram showing the pn junction according to the third embodiment and an energy band of the pn junction at the time of forward direction bias.
[Fig. 16] A diagram showing the pn junction according to the third embodiment and an energy band of the pn junction at the time of reverse direction bias.
[Fig. 17] A schematic diagram for explaining the movement of electron holes through a gateway of a p-channel in p-type zinc-substituted cytochrome c used as a protein semiconductor constituting the pn junction according to the third embodiment.
[Fig. 18] A schematic diagram for explaining the movement of electron holes through a gateway of a p-channel in p-type zinc-substituted cytochrome c used as a protein semiconductor constituting the pn junction according to the third embodiment.
[Fig. 19] A schematic diagram for explaining the movement of electron holes through a gateway of an n-channel in p-type zinc-substituted cytochrome b₅₆₂ used as a protein semiconductor constituting the pn junction according to the third embodiment.
[Fig. 20] A schematic diagram for explaining the movement of electron holes through a gateway of an n-channel in p-type zinc-substituted cytochrome b₅₆₂ used as a protein semiconductor constituting the pn junction according to the third embodiment.
[Fig. 21] A schematic diagram showing a light emission element according to a fourth embodiment.
[Fig. 22] A schematic diagram showing an n-type quantum cascade laser according to a fifth embodiment.
[Fig. 23] A schematic diagram showing a p-type quantum cascade laser according to the fifth embodiment.
[Fig. 24] A schematic diagram showing a bulk-heterojunction type photoelectric conversion element according to a sixth embodiment.
[Fig. 25] A schematic diagram showing a structural example of the bulk-heterojunction type photoelectric conversion element according to the sixth embodiment.
[Fig. 26] A schematic diagram showing another structural example of the bulk-heterojunction type photoelectric conversion element according to the sixth embodiment.
[Fig. 27] An exemplary energy band diagram of the bulk-heterojunction type photoelectric conversion element according to the sixth embodiment.
[Fig. 28] Another exemplary energy band diagram of the bulk-heterojunction type photoelectric conversion element according to the sixth embodiment.
[Fig. 29] An energy band diagram of a protein semiconductor constituting an electric field detection element according to a ninth embodiment.
[Fig. 30] A schematic diagram showing a photo sensor according to a tenth embodiment.
[Fig. 31] A schematic diagram showing an inverter circuit according to an eleventh embodiment.

### Mode(s) for Carrying Out the Invention

Hereinafter, a mode for carrying out the invention (hereinafter, referred to as "embodiment") will be described. It should be noted that a description will be given in the following order.
1. First Embodiment (method of controlling conductivity type of protein semiconductor)
2. Second Embodiment(method of manufacturing protein semiconductor and protein semiconductor)
3. Third Embodiment (method of manufacturing pn junction and pn junction)
4. Fourth Embodiment (light emission element)
5. Fifth Embodiment (quantum cascade laser)
6. Sixth Embodiment (bulk-heterojunction type photoelectric conversion element)
7. Seventh Embodiment (electric field detection element)
8. Eighth Embodiment (bipolar transistor)
9. Ninth Embodiment (thyristor)
10. Tenth Embodiment (photo sensor)
11. Eleventh Embodiment (inverter circuit)

### <1. First Embodiment>

### [Method of Controlling Conductivity Type of Protein Semiconductor]

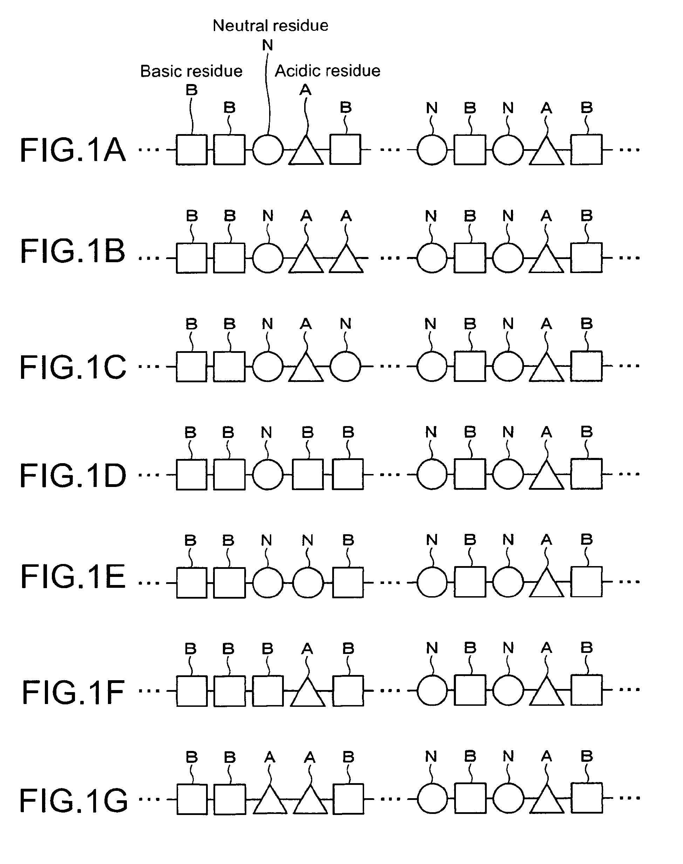

Fig. 1A shows an example of a protein semiconductor.

As shown in Fig. 1A, the protein semiconductor is obtained by bonding a basic amino acid residue (hereinafter, referred to as, simply, basic residue) B, an acidic amino acid residue (hereinafter, referred to as, simply, acidic residue) A, and a neutral amino acid residue (hereinafter, referred to as, simply, neutral residue) N together by peptide bonds. Arrangement order and number of the basic residue B, the acidic residue A, and the neutral residue N shown in Fig. 1A are temporarily set, and the arrangement order and number differ depending on the protein semiconductor. For the sake of convenience, the basic residue B is represented by a rectangular shape, the acidic residue A is represented by a triangular shape, and the neutral residue N is represented by a circular shape. The basic residue B is a residue of lysine (Lys), arginine (Arg), or histidine (His). The acidic residue A is a residue of glutamic acid (Glu) or aspartic acid (Asp). The neutral residue N is a residue of serine (Ser), threonine (Thr), asparagine (Asn), glutamine (Gln), alanine (Ala), cysteine (Cys), glycine (Gly), isoleucine (Ile), leucine (Leu), methionine (Met), phenylalanine (Phe), proline (Pro), tryptophan (Trp), tyrosine (Tyr), or valine (Val).

A method of controlling properties of the protein semiconductor shown in Fig. 1A will be described. 1. One or More of Basic Residue B in Protein Semiconductor Shown in Fig. 1A Are Substituted with Acidic Residue A.

An example thereof is shown in Fig. 1B. As shown in Fig. 1B, the fifth basic residue B from the left in the protein semiconductor shown in Fig. 1A is substituted with the acidic reside A. Accordingly, total amount of charge of the amino acid residues in the protein semiconductor shown in Fig. 1B is changed, specifically, reduced with respect to the total amount of charge of the amino acid residues in the protein semiconductor shown in Fig. 1A. As a result, for example, although the protein semiconductor shown in Fig. 1A shows a p-type photocurrent response, the protein semiconductor shown in Fig. 1B is changed to show an n-type photocurrent response.

2. One or More of Basic Residue B in Protein Semiconductor Shown in Fig. 1A Are Substituted with Neutral Residue N.

An example thereof is shown in Fig. 1C. As shown in Fig. 1C, the fifth basic residue B from the left in the protein semiconductor shown in Fig. 1A is substituted with the neutral residue N. Accordingly, total amount of charge of the amino acid residues in the protein semiconductor shown in Fig. 1C is changed, specifically, reduced with respect to the total amount of charge of the amino acid residues in the protein semiconductor shown in Fig. 1A. As a result, for example, although the protein semiconductor shown in Fig. 1A shows a p-type photocurrent response, the protein semiconductor shown in Fig. 1C is changed to show an n-type photocurrent response.

3. One or More of Acidic Residue A in Protein Semiconductor Shown in Fig. 1A Are Substituted with Basic Residue B.

An example thereof is shown in Fig. 1D. As shown in Fig. 1D, the fourth acidic residue A from the left in the protein semiconductor shown in Fig. 1A is substituted with the neutral residue N. Accordingly, total amount of charge of the amino acid residues in the protein semiconductor shown in Fig. 1D is changed, specifically, increased with respect to the total amount of charge of the amino acid residues in the protein semiconductor shown in Fig. 1A. As a result, for example, although the protein semiconductor shown in Fig. 1A shows a p-type photocurrent response, the protein semiconductor shown in Fig. 1D is changed to show an n-type photocurrent response.

4. One or More of Acidic Residue A in Protein Semiconductor Shown in Fig. 1A Are Substituted with Neutral Residue N.

An example thereof is shown in Fig. 1E. As shown in Fig. 1E, the fourth acidic residue A from the left in the protein semiconductor shown in Fig. 1A is substituted with the neutral residue N. Accordingly, total amount of charge of the amino acid residues in the protein semiconductor shown in Fig. 1E is changed, specifically, increased with respect to the total amount of charge of the amino acid residues in the protein semiconductor shown in Fig. 1A. As a result, for example, although the protein semiconductor shown in Fig. 1A shows a p-type photocurrent response, the protein semiconductor shown in Fig. 1E is changed to show an n-type photocurrent response.

5. One or More of Neutral Residue N in Protein Semiconductor Shown in Fig. 1A Are Substituted with Basic Residue B.

An example thereof is shown in Fig. 1F. As shown in Fig. 1F, the third neutral residue N from the left in the protein semiconductor shown in Fig. 1A is substituted with the basic residue B. Accordingly, total amount of charge of the amino acid residues in the protein semiconductor shown in Fig. 1F is changed, specifically, increased with respect to the total amount of charge of the amino acid residues in the protein semiconductor shown in Fig. 1A. As a result, for example, although the protein semiconductor shown in Fig. 1A shows a p-type photocurrent response, the protein semiconductor shown in Fig. 1F is changed to show an n-type photocurrent response.

6. One or More of Neutral Residue N in Protein Semiconductor Shown in Fig. 1A Are Substituted with Acidic Residue A.

An example thereof is shown in Fig. 1G. As shown in Fig. 1G, the third neutral residue N from the left in the protein semiconductor shown in Fig. 1A is substituted with the acidic residue A. Accordingly, total amount of charge of the amino acid residues in the protein semiconductor shown in Fig. 1G is changed, specifically, reduced with respect to the total amount of charge of the amino acid residues in the protein semiconductor shown in Fig. 1A. As a result, for example, although the protein semiconductor shown in Fig. 1A shows a p-type photocurrent response, the protein semiconductor shown in Fig. 1G is changed to show an n-type photocurrent response.

7. One or More of Basic Residue B in Protein Semiconductor Shown in Fig. 1A Are Neutralized or Acidified by Chemical Modification. Alternatively, one or more of the acidic residue A in the protein semiconductor shown in Fig. 1A are neutralized or basified by chemical modification. Alternatively, one or more of the neutral residue N in the protein semiconductor shown in Fig. 1A are acidified or basified by chemical modification.

For example, the fifth basic residue B from the left in the protein semiconductor shown in Fig. 1A is chemically modified to turn it into a neutral residue or an acidic residue. Accordingly, total amount of charge of the amino acid residues in the protein semiconductor is changed, specifically, reduced with respect to the total amount of charge of the amino acid residues in the protein semiconductor shown in Fig. 1A. As a result, for example, although the protein semiconductor shown in Fig. 1A shows a p-type photocurrent response, the protein semiconductor is changed to show an n-type photocurrent response.

Alternatively, for example, the fourth acidic residue A from the left in the protein semiconductor shown in Fig. 1A is chemically modified to turn it into a neutral residue or a basic residue. Accordingly, total amount of charge of the amino acid residues in the protein semiconductor is changed, specifically, increased with respect to the total amount of charge of the amino acid residues in the protein semiconductor shown in Fig. 1A. As a result, for example, although the protein semiconductor shown in Fig. 1A shows an n-type photocurrent response, the protein semiconductor is changed to show a p-type photocurrent response.

Alternatively, for example, the third neutral residue N from the left in the protein semiconductor shown in Fig. 1A is chemically modified to turn it into a basic residue or an acidic residue. Accordingly, total amount of charge of the amino acid residues in the protein semiconductor is changed, specifically, increased or reduced with respect to the total amount of charge of the amino acid residues in the protein semiconductor shown in Fig. 1A. As a result, for example, although the protein semiconductor shown in Fig. 1A shows an n-type photocurrent response, the protein semiconductor is changed to show a p-type photocurrent response.

Chemical modification methods are exemplified as follows.

### ·Acetylation of lysine residue (Lys)

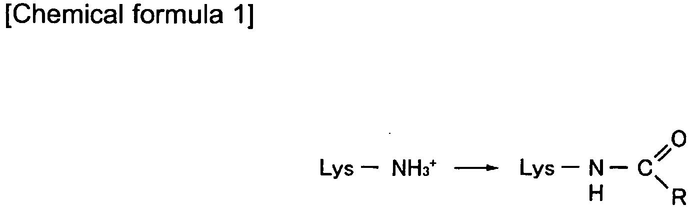

### •Succinylation of serine residue (Ser)

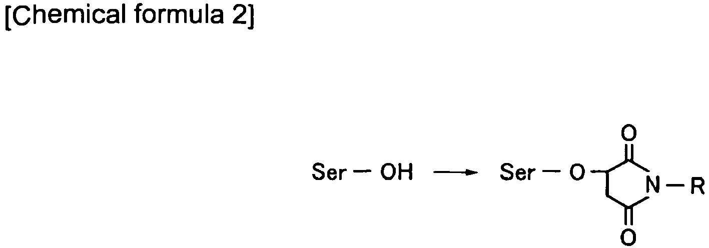

### •Succinylation of threonine residue (Thr)

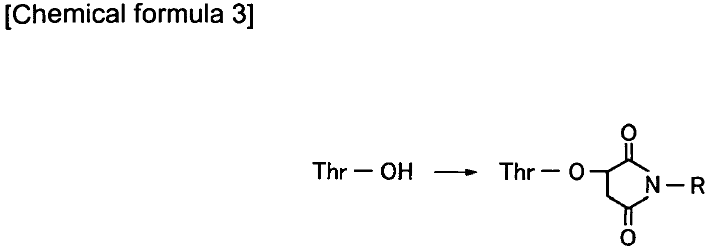

### •Disulfidation of cysteine residue (Cys)

[Chemical formula 4] Cys-S → Cys- S- S-R

[Chemical formula 5] Cys-S → Cys- S-R

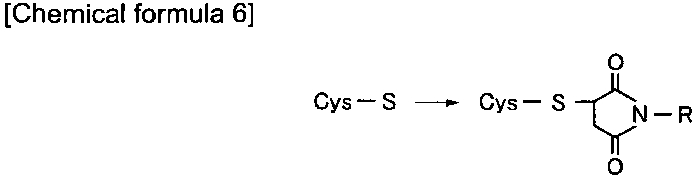

### •Esterification of aspartic acid residue (Asp)

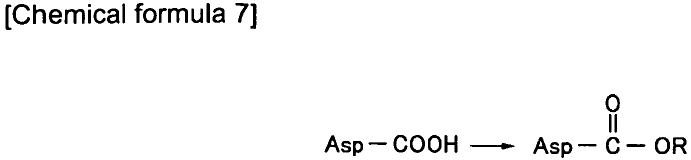

### ·Amidation of aspartic acid residue (Asp)

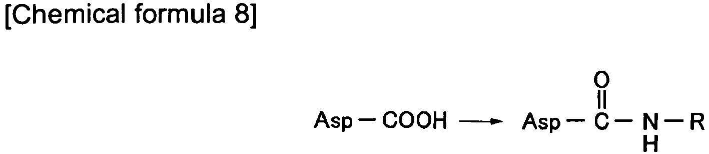

### •Esterification of glutamine residue (Glu)

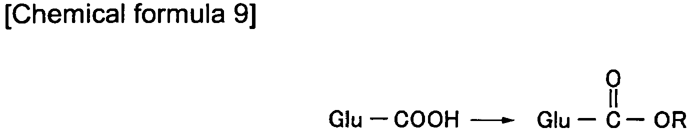

### •Amidation of glutamine residue (Gln)

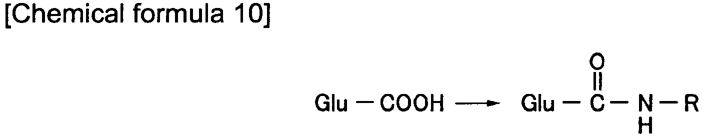

### •Phosphorylation of tyrosine residue (Tyr)

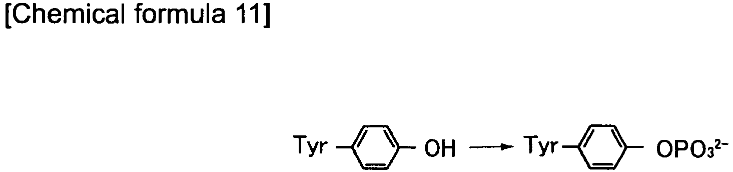

### ·Phosphorylation of serine residue (Ser)

[Chemical formula 12] Ser-OH → Ser-OPO₃²⁻

8. Polarity of Medium Surrounding Protein Semiconductor Shown Fig. 1A Is Controlled.

The medium surrounding the protein semiconductor may be any one of liquid, gel, and solid materials.

For example, the protein semiconductor shown Fig. 1A is surrounded by a buffer solution having a high degree of basicity, a basic solution, a basic polymer, or the like. Alternatively, for example, the protein semiconductor shown Fig. 1A is surrounded by a buffer solution having a high degree of acidity, an acidic solution, an acidic polymer, or the like. Accordingly, for example, although the protein semiconductor shown in Fig. 1A shows a p-type photocurrent response, this protein semiconductor is changed to show an n-type photocurrent response. Alternatively, although the protein semiconductor shown in Fig. 1A shows an n-type photocurrent response, this protein semiconductor is changed to show a p-type photocurrent response.

### [Example 1]

Zinc-substituted cytochrome c shows a p-type photocurrent response.

By substituting one or more of basic residues in the zinc-substituted cytochrome c with an acidic residue or a neutral residue, the p-type photocurrent response is converted into an n-type photocurrent response.

The amino acid sequence (one-character code) of the zinc-substituted cytochrome c is as follows. The number of amino acid residues in the zinc-substituted cytochrome c is 104.
GDVEKGKKIF VQKCAQCHTV EKGGKHKTGP
NLHGLFGRKT GQAPGFTYTD ANKNKGITWK
EETLMEYLEN PKKYIPGTKM IFAGIKKKTE
REDLIAYLKK ATNE

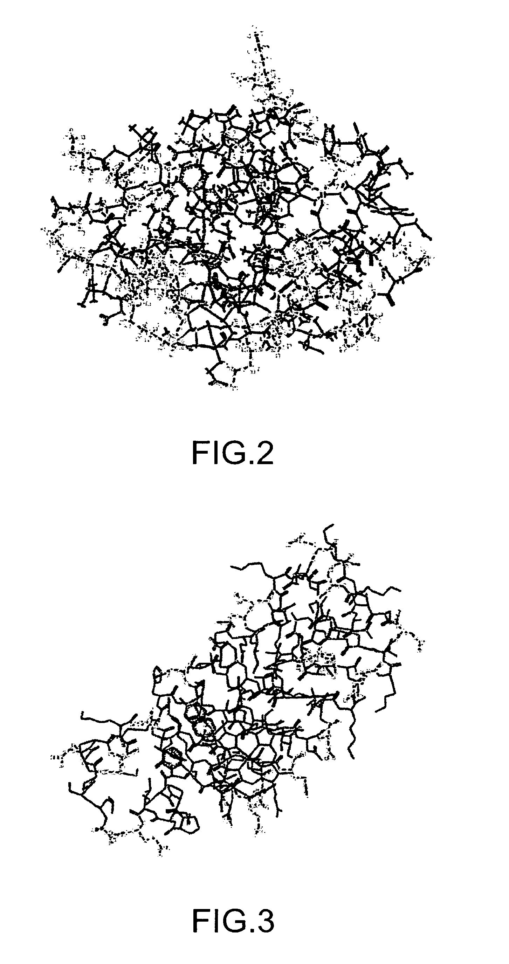

Fig. 2 shows positions of basic residues in the zinc-substituted cytochrome c. The basic residues in the zinc-substituted cytochrome c are lysine (K in one-character code and Lys in three-character code) and arginine (R in one-character code and Arg in three-character code), and the residue numbers are as follows.
·Lysine 5, 7, 8, 13, 22, 25, 27, 39, 53, 55, 60, 72, 73, 79, 86, 87, 88, 99, 100
·Arginine 38, 91

### [Example 2]

The zinc-substituted cytochrome b₅₆₂ shows a p-type photocurrent response.

By substituting one or more of acidic residues in the zinc-substituted cytochrome b₅₆₂ with a basic residue or a neutral residue, the p-type photocurrent response is converted into the n-type photocurrent response.

The amino acid sequence (one-character code) of the zinc-substituted cytochrome b₅₆₂ is as follows. The number of amino acid residues in the zinc-substituted cytochrome b₅₆₂ is 106.

ADLEDNMETL NDNLKVIEKA DNAAQVKDAL
TKMRAAALDA QKATPPKLED KSPDSPEMKD
FRHGFDILVG QIDDALKLAN EGKVKEAQAA
AEQLKTTRNA YHQKYR

Fig. 3 shows positions of basic residues in the zinc-substituted cytochrome b₅₆₂. The acidic residues in the zinc-substituted cytochrome b₅₆₂ are glutamic acid and aspartic acid, and the residue numbers are as follows.
·Glutamic acid 4, 8, 18, 49, 57, 81, 86, 92
·Aspartic acid 2, 5, 12, 21, 28, 39, 50, 54, 60, 66, 73, 74

### [Example 3]

By neutralizing or acidifying one or more of basic residues in the zinc-substituted cytochrome c by chemical modification, the p-type photocurrent response is converted into the n-type photocurrent response.

The positions of the basic residues in the zinc-substituted cytochrome c are shown in Fig. 2, and the residue numbers of lysine and arginine, which are basic residues, are as described above.

For example, by introducing a neutral residue as R with acetylation of a lysine residue being a basic residue, the basic residues are converted into neutral residues. Specifically, for example, by introducing an uncharged substitution group such as a methyl group and an ethyl group as R, the basic residues are converted into neutral residues. Moreover, in the case where the basic residues are acidified, an acidic group such as a sulfonyl methylene group and a carbonyl methylene group is introduced as R.

### [Example 4]

The zinc-substituted cytochrome b₅₆₂ shows the n-type photocurrent response.

By neutralizing or basifying one or more of acidic residues in the zinc-substituted cytochrome b₅₆₂ by chemical modification, the n-type photocurrent response is converted into the p-type photocurrent response.

The positions of the acidic residues in the zinc-substituted cytochrome b₅₆₂ are shown in Fig. 3, and the residue numbers of glutamic acid and aspartic acid, which are acidic residues, are as described above.

For example, by introducing a neutral residue as R with esterification or amidation of glutamic acid or aspartic acid, which is an acidic residue, the acidic residues are converted into neutral residues. Specifically, for example, an uncharged substitution group such as a methyl group and an ethyl group is introduced as R. Alternatively, in the case where the acidic residues are converted into basic residues, a basic group is introduced as R.

### [Example 5]

By acidifying one or more of neutral residues in the zinc-substituted cytochrome c by chemical modification, the p-type photocurrent response is converted into the n-type photocurrent response. For example, threonine and tyrosine having OH groups, which are neutral residues, are acidified by phosphorylation.

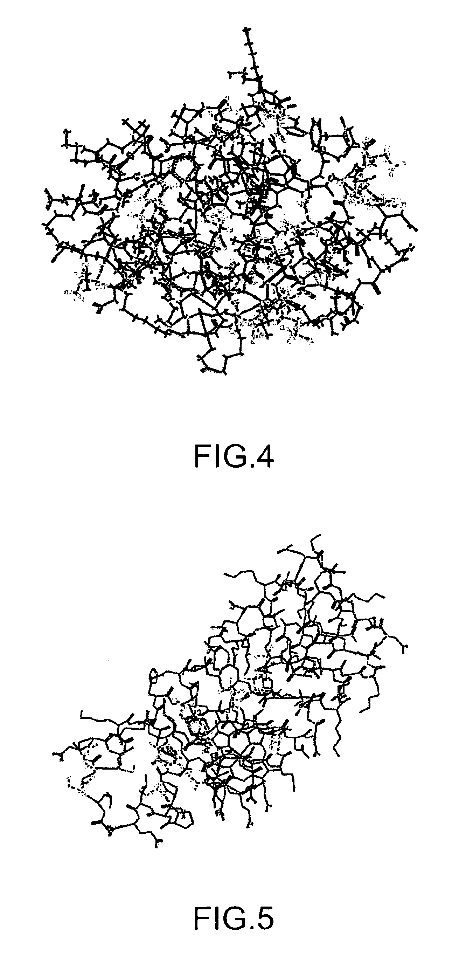

Fig. 4 shows the positions of threonine and tyrosine, which are neutral residues having OH groups, in the zinc-substituted cytochrome c, and the residue numbers of threonine and tyrosine are as follows.
- Threonine19, 28, 40, 47, 49, 58, 63, 78, 89, 102
- Tyrosine 48, 67, 74, 97

### [Example 6]

The zinc-substituted cytochrome b₅₆₂ shows the n-type photocurrent response.

By basifying one or more of neutral residues in the zinc-substituted cytochrome b₅₆₂ by chemical modification, the n-type photocurrent response is converted into the p-type photocurrent response. For example, serine, threonine, and tyrosine having OH groups, which are neutral residues, are acidified byphosphorylation.

Fig. 5 shows the positions of serine, threonine, and tyrosine, which are neutral residues having OH groups, in the zinc-substituted cytochrome b₅₆₂, and the residue numbers are as follows.
·Threonine9, 31, 44, 96, 97
·Tyrosine 101, 105
·Serine 52, 55

### [Example 7]

By surrounding the zinc-substituted cytochrome c with a buffer solution having a high degree of basicity, a basic solution, or a basic polymer, the p-type photocurrent response is converted into the n-type photocurrent response.

### [Example 8]

By surrounding the zinc-substituted cytochrome b₅₆₂ with a buffer solution having a high degree of acidity, an acidic solution, or an acidic polymer, the n-type photocurrent response is converted into the p-type photocurrent response.

### [Method of Preparing Zinc-Substituted Cytochrome b₅₆₂]

Here, a method of preparing zinc-substituted cytochrome b₅₆₂ and the properties thereof will be described.

### a. Method of Expressing/Purifying Cytochrome b₅₆₂ Derived from Escherichia Coli

A plasmid (Cyt-b562/pKK223-3) to which a structural gene of cytochrome b₅₆₂ derived from Escherichia coli is introduced is prepared, and is transformed into Escherichia Coli strain JM109. The expressing/purifying method was performed according to Non-Patent Document 2.

A preculture solution overnight cultured in 100mL of LB-Amp medium at 37°C was transferred to 4L(2Lx2) of Terrific broth and was cultured at 37°C for 5 to 6 hours. Zero point two mM of IPTG is added thereto, and the mixture thus obtained was cultured for 18 hours at 25°C. Thus, 70 g of red bacterial cells was obtained. The frozen bacterial cells are suspended in 200 mL of 10mM of Tris-HCl (pH 8.0) containing 1mM of EDTA, 1mM of PMSF, 0.2mg/mL of Lysozyme, DTT (as appropriate), and DNase (as appropriate), and the cells were destroyed by ultrasonic waves.

Two M of phosphate was added to the centrifugal supernatant and the pH was adjusted to 4.55. Then, centrifugal precipitation of unnecessary protein was performed. The sample thus obtained was purified with CM52 negative ion exchange column chromatography (80mL of column volume, 50 to 150 mM of KCl, linear gradient/50mM of potassium phosphate (pH 4.55)), and Sephadex G50 Fine gel-filtration chromatography (480mL of column volume, 50mM of Tris-HCl, 0.1mM of EDTA, pH 8.0), and thus about 80 mg of cytochrome b₅₆₂ was obtained.

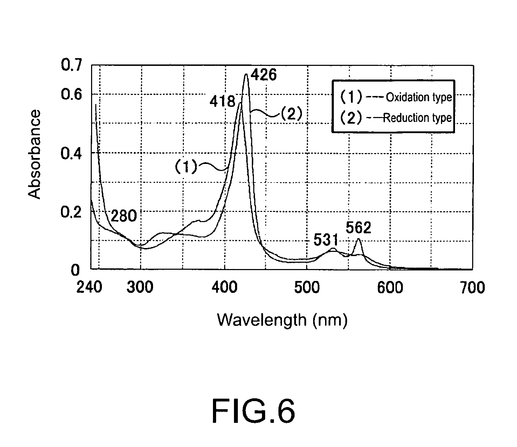

Fig. 6 shows the absorption spectrum of the purified cytochrome b₅₆₂. The measurement was performed in a state where the purified cytochrome b₅₆₂ was immersed in 10 mM of sodium phosphate buffer solution (pH 7.0). As shown in Fig. 6, in the purified state, the cytochrome b₅₆₂ was oxidation type having absorption peaks at 418 nm and 532 nm. A small amount of dithionite is added to the buffer solution to make the cytochrome b₅₆₂ be reduction type. Then, absorption peaks at 426 nm, 531 nm, and 562 nm were confirmed.

The amino acid sequence of the cytochrome b₅₆₂ thus obtained is as follows. In the amino acid sequence, as will be described later, an underlined methionine 7, an underlined histidine 102, and an underlined isoleucine 17 of ligands of heme play an important role.

ADLEDNMETL NDNLKVIEKA DNAAQVKDAL TKMRAAALDA
QKATPPKLED KSPDSPEMKD FRHGFDILVG QIDDALKLAN EGKVKEAQAA
AEQLKTTRNA YHQKYR

### b. Immobilization of Cytochrome b₅₆₂ to Gold Drop Electrode

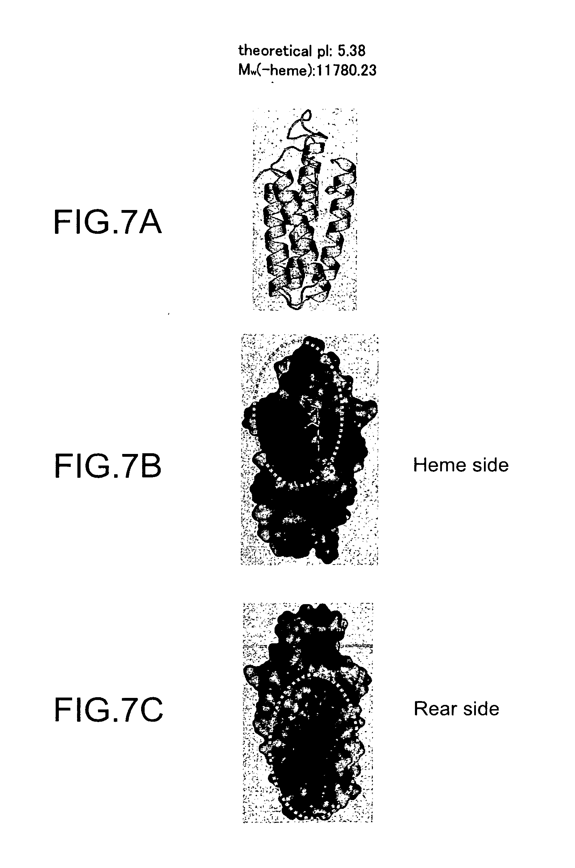

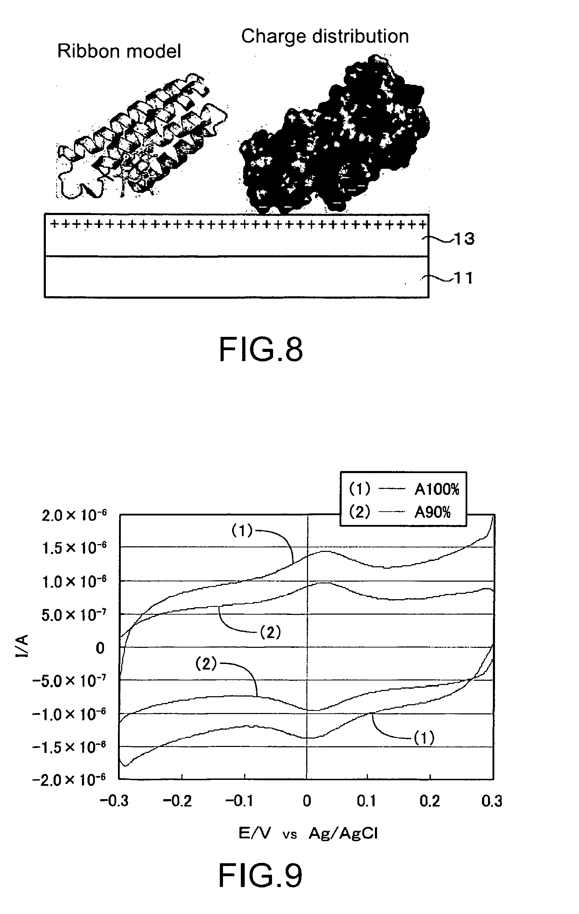

A crystal structure of the cytochrome b₅₆₂ determined by X-ray crystal structure analysis in 1979 (see Non-Patent Document3) is shown in Fig. 7A, B, and C. Here, Fig. 7A shows a ribbon model, and the heme and ligand amino acids thereof are represented by a stick model. Fig. 7B shows charge distribution at the time when the cytochrome b₅₆₂ is in the same direction as that in Fig. 7A, and a portion surrounded by broken lines having an elliptical shape is a most strongly negatively charged heme-propionic acid exposed surface (the same holds true for Fig. 7C). Fig. 7C shows charge distribution of the state (rear side of the cytochrome b₅₆₂ in the state shown in Fig. 7B) of the cytochrome b₅₆₂ rotated about the vertical axis by 180 degrees from the state shown in Fig. 7B. As shown in Fig. 7A, B, and C, the cytochrome b₅₆₂ has a 4-helix bundle structure and a molecule of heme being a prosthetic group. The propionic acid of the heme is exposed by sticking out from the molecule. From the charge distribution shown in Fig. 7B, it can be seen that the heme-propionic acid site has a strong negative charge. Therefore, if a surface of the gold electrode has a positive charge, it is possible to adsorb the cytochrome b₅₆₂ into the gold electrode at the heme-propionic acid site. The schematic diagram is shown in Fig. 8 (only heme is represented by a stick model). In this example, a self-assembled monolayer 13 having a positive charge on its outermost surface is formed on a gold electrode 11, and an electrostatic attractive force exerted between a positive charge on the outermost surface of the self-assembled monolayer 13 and a positive charge of the heme-propionic acid site of the cytochrome b₅₆₂ causes the cytochrome b₅₆₂ to adsorb to the self-assembled monolayer 13.

As the gold electrode, a gold drop electrode having a diameter of 2 mm was formed.

The gold drop electrode was washed with hot concentrated sulfuric acid (120°C), and the roughness of the surface of the gold drop electrode was increased in redox processes in sulfuric acid. The gold drop electrode was immersed in 0.1mM of 11-aminoundecanethiol (H₂ N-C₁₁-SH)/ethanol solution at room temperature for 16 hours or more, and an H₂ N-C₁₁-SH film was formed on the surface of the gold drop electrode as the self-assembled monolayer 13. Thus, compressed air is applied to the gold drop electrode on which the H₂ N-C11-SH film was formed, and the gold drop electrode was dried. After that, the gold drop electrode was soaked in 60µL of 50µM of cytochrome b₅₆₂ /4.4mM of potassium phosphate (pH 7.2) solution, and is incubated at 4°C all day long.

Fig. 9 shows a cyclic voltammogram measured by immersing the incubated gold drop electrode in 10 mM of sodium phosphate (pH 7.0). The potential scan rate is 1 V/s. As shown in Fig. 9, an adsorption-type cyclic voltammogram was obtained. The effective surface area of the cytochrome b₅₆₂ on the surface of the gold drop electrode was 1.7±0.6 pmol/cm², the oxidation-reduction potential was -4±11 mV vs Ag/AgCl, and the electron transfer rate constant between the cytochrome b₅₆₂ and the gold drop electrode was 90+12 s⁻¹. The similar adsorption effect can be obtained by mixing the 11-aminoundecanethiol formed on the surface of the gold drop electrode with 0 to 10% of hydroxyundecanethiol. Fig. 9 shows a cyclic voltammogram obtained by mixing the 11-aminoundecanethiol with 10% of hydroxyundecanethiol.

### c. Preparation of Zinc-Substituted Cytochrome b₅₆₂

Because a method of preparing the zinc-substituted cytochrome b₅₆₂ has been reported by Hamachi et al. (Non-Patent Document 4), the zinc-substituted cytochrome b₅₆₂ was prepared according thereto.

First, 1 M of hydrochloric acid is added to 3 mL of cytochrome b₅₆₂ aqueous solution (33 µM), and the pH is adjusted to 2 to 3. To the cytochrome b₅₆₂ aqueous solution thus obtained, 3 mL of 2-butanone, which has been water-cooled, is added, the mixture was gently agitated, heme was extracted from the cytochrome b₅₆₂, and the butanone layer was removed by pipetting (see Non-Patent Document 5). The extraction operation was repeated until the butanone layer shows no color. A slight amount of 1 M of Tris-HCl (pH 8.0) is added to the aqueous solution in which the extraction operation of heme has been repeated, and the pH is adjusted to 7 to 8. After that, dialysis (2L×5 times) is performed against ultrapure water, and thus apocytochrome b₅₆₂ was obtained.

Zinc protoporphyrin IX (ZnPP) was dissolved in dimethyl sulfoxide, and 2 equal amount of the mixture thus obtained was added to the above-mentioned apocytochrome b₅₆₂. A protein fraction is collected from the mixture by using a Biogel P10 desalting column equilibrated in advance with 50 mM of Tris-HCl (pH 8.0) and 0.1 mM of EDTA, and the purified zinc-substituted cytochrome b₅₆₂ (Zn-Cyt b₅₆₂) was obtained.

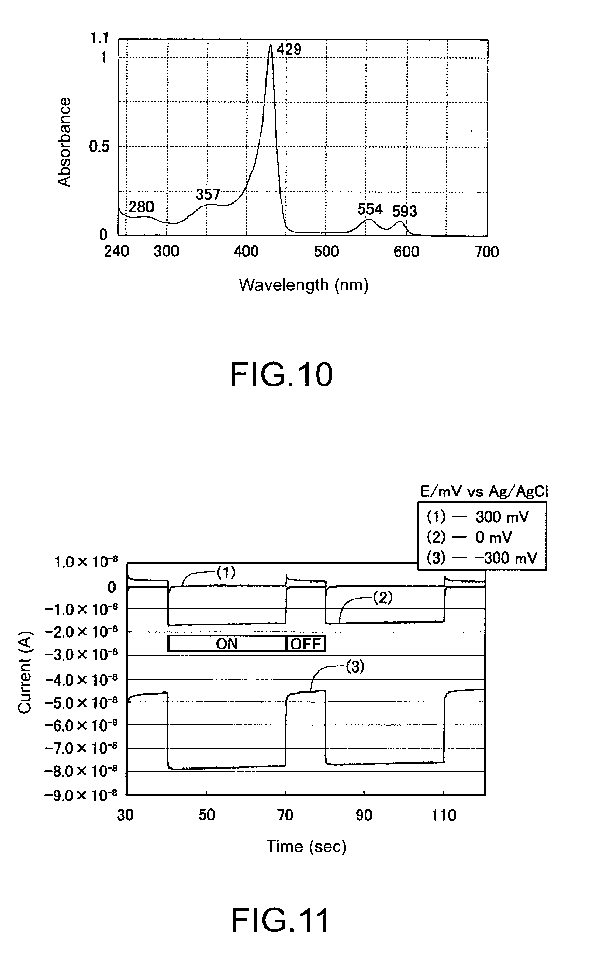

Fig. 10 shows the absorption spectrum of the obtained zinc-substituted cytochrome b₅₆₂. The measurement was performed in a state where the zinc-substituted cytochrome b₅₆₂ was immersed in 10 mM of sodium phosphate buffer solution (pH 7.0). As shown in Fig. 10, absorption peaks were present at 280 nm, 357 nm, 429 nm, 554 nm, and 593 nm, and the positions thereof corresponded to those described in Non-Patent Document 4. Moreover, the ratio of absorbance at the wavelength of 429 nm to that at 554 nm (A429/A554) was 11.05.

### d. Immobilization of Zinc-Substituted Cytochrome b₅₆₂ to Gold Drop Electrode and Photocurrent Measurement

As the gold electrode 11, a gold drop electrode having a diameter of 2 mm was formed.

The gold drop electrode was washed with hot concentrated sulfuric acid (120°C), and the roughness of the surface of the gold drop electrode was increased in redox processes in sulfuric acid. The gold drop electrode was immersed in 0.1 mM of 11-aminoundecanethiol (H₂ N-C₁₁-SH)/ethanol solution at room temperature for 16 hours or more, and an H₂ N-C₁₁-SH film was formed on the surface of the gold drop electrode as the self-assembled monolayer 13. Thus, compressed air is applied to the gold drop electrode on which the H₂ N-C11-SH film was formed, and the gold drop electrode was dried. After that, the gold drop electrode was soaked in 60 µL of 50 µM of zinc-substituted cytochrome b₅₆₂ /4.4 mM of potassium phosphate (pH 7.2) solution, and is incubated at 4°C all day long.

The photocurrent measurement was performed in 10 mM of nitrogen purged sodium phosphate (pH 7.0) by using Ag/AgCl as a reference electrode and a Pt mesh electrode as a counter electrode.

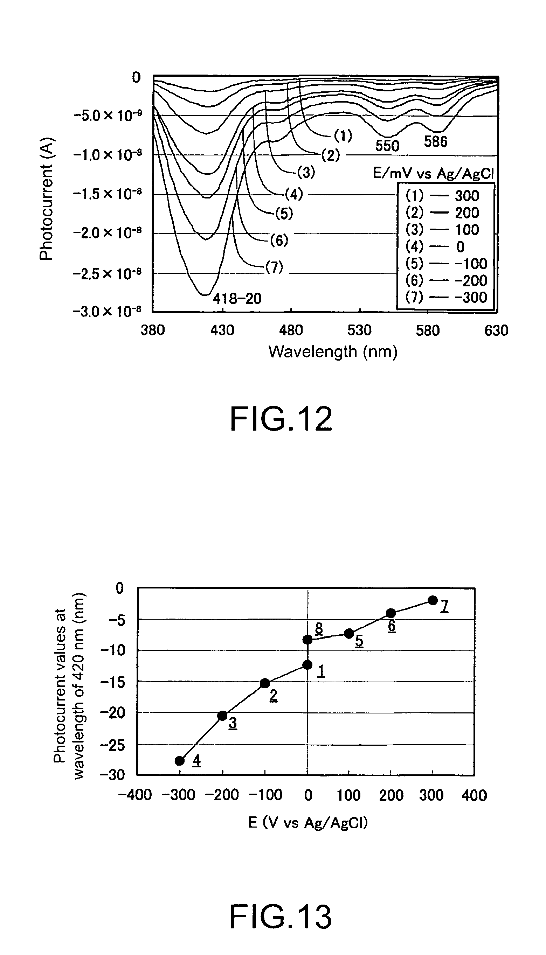

The results of the photocurrent measurement (photocurrent real-time waveforms) with the bias voltage of 300 mV, 0 mV, and -300 mV are shown in Fig. 11. Fig. 11 shows current values plotted against time, which are obtained when light at the wavelength of 420 nm is irradiated for 30 seconds and is turned off for 10 seconds. As shown in Fig. 11, in all the range of the bias voltage, cathodic photocurrent was observed. Fig. 12 shows a photocurrent action spectrum. As shown in Fig. 12, wavelengths showing the peak current are 418 to 420 nm, 550 nm, and 586 nm, and are significantly different from absorption maximum wavelengths of 429 nm, 554 nm, and 593 nm in the solution ultraviolet-visible absorption spectrum of the zinc-substituted cytochrome b₅₆₂ shown in Fig. 13. Moreover, the ratio of the photocurrent at the wavelength of 418 to 420 nm to that at 550 nm is 3.7, and is significantly below the ratio of the photocurrent in the absorption spectrum shown in Fig. 10, i.e., 11.05. Fig. 13 shows a graph of photocurrent values at the wavelength of 420 nm plotted against potential E. In Fig. 13, numbers added to the current-voltage curve represent the order of the obtained data. According to Patent Document 1, in the case where the zinc-substituted cytochrome c is immobilized to the gold electrode, a threshold is about - 100 mV(vs Ag/AgCl), and the inversion of the photocurrent is observed around the boundary of the potential. On the other hand, as shown in Fig. 13, in the case of the zinc-substituted cytochrome b₅₆₂, it cannot be seen. Moreover, even if potassium ferrocyanide is added thereto, the photocurrent is not enhanced. This is different from Patent Document 1.

As described above, according to the first embodiment, by controlling total amount of charge in amino acid residues in a protein semiconductor by various methods, it is possible to easily control a conductivity type of the protein semiconductor.

### <2. Second Embodiment>

### [Method of Manufacturing Protein Semiconductor and Protein semiconductor]

In a second embodiment, a protein semiconductor having a desired conductivity type, specifically, p-type protein semiconductor, n-type protein semiconductor, or i-type protein semiconductor, is manufactured by using the method of controlling a conductivity type of a protein semiconductor according to the first embodiment

According to the second embodiment, it is possible to easily manufacture the p-type protein semiconductor, n-type protein semiconductor, and i-type protein semiconductor. Therefore, at least a part of an element constituting an electronic circuit can be formed by using the p-type protein semiconductor, n-type protein semiconductor, i-type protein semiconductor, or a pn junction obtained by joining the p-type protein semiconductor and the n-type protein semiconductor.

### <3. Third Embodiment>

### [Pn Junction and Method of Manufacturing Pn Junction]

In a third embodiment, a pn junction is manufactured by joining the p-type protein semiconductor and the n-type protein semiconductor, which are manufactured in the second embodiment.

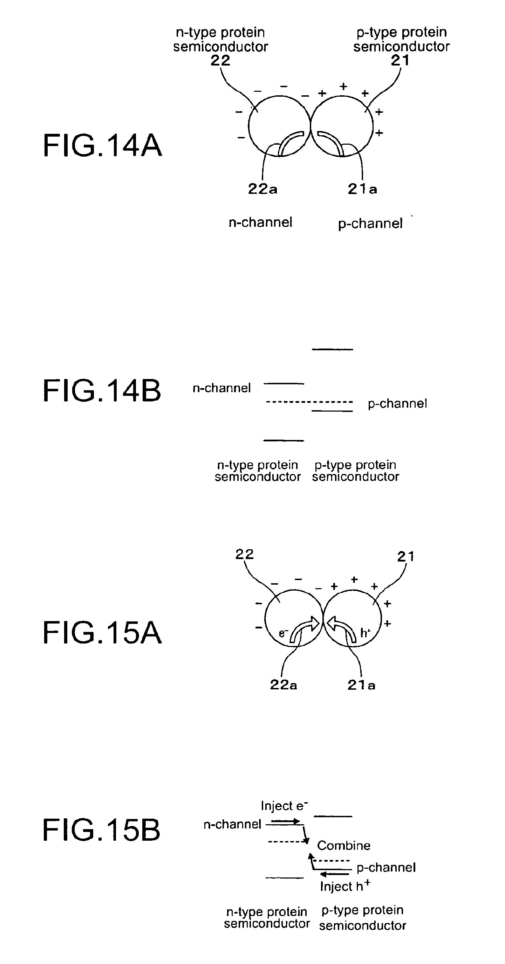

Fig. 14A shows the pn junction manufactured in this way. As shown in Fig. 14A, the pn junction is obtained by joining a p-type protein semiconductor 21 and an n-type protein semiconductor 22. As described above, the p-type protein semiconductor 21 and the n-type protein semiconductor 22 are manufactured by controlling total amount of charge in amino acid residues. However, the p-type protein semiconductor 21 and the n-type protein semiconductor 22 are characterized by the polarity of surface charges. Specifically, as shown in Fig. 14A, the surface of the p-type protein semiconductor 21 is positively (+) charged, and the surface of the n-type protein semiconductor 22 is negatively (-) charged. In other words, by controlling the surface charge of the protein semiconductor, it is possible to control the position of the molecular orbital and therefore the energy band.

Fig. 14B shows the energy band of the pn junction at the time of zero bias. As shown in Fig. 14B, a p-channel 21 to be a movement path of electron holes is formed by the molecular orbital on the p-type protein semiconductor 21, and an n-channel 22a to be a movement path of electrons is formed by the molecular orbital on the n-type protein semiconductor 22. The energy of the n-channel 22a is higher than that of the p-channel 21a.

Fig. 15A shows the pn junction at the time when forward direction bias is applied. Moreover, Fig. 15B shows the energy band of the pn junction at the time when forward direction bias is applied. As shown in Fig. 15A and B, when forward direction bias is applied, an electron hole (h⁺) is moved from the p-channel 21a to the joining portion of the pn junction, and an electron (e⁻) is moved from the n-channel 22a. Accordingly, current is flown through the pn junction, and a part of electrons and electron holes is recombined.

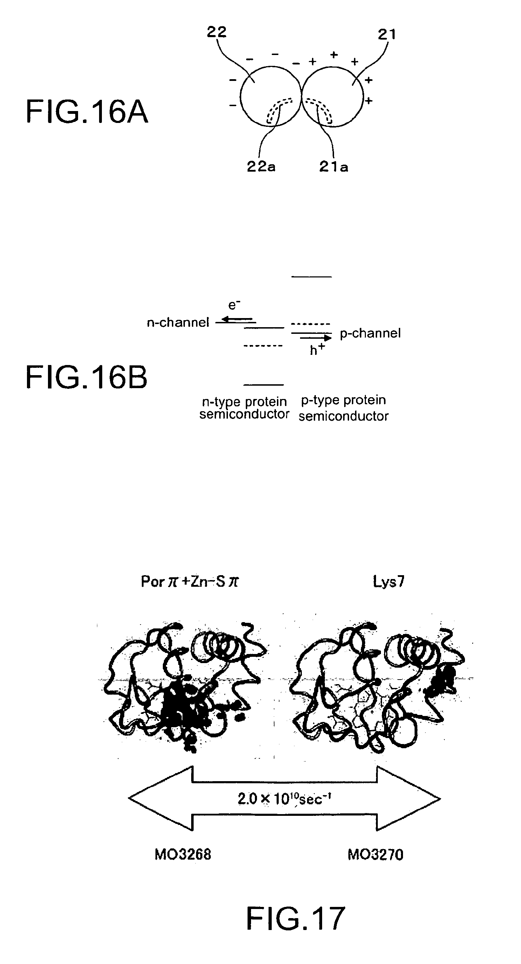

Fig. 16A shows the pn junction at the time when reverse direction bias is applied. Moreover, Fig. 16B shows the energy band of the pn junction at the time when reverse direction bias is applied. As shown in Fig. 16A and B, when reverse direction bias is applied, electron holes and electrons are moved away from the joining portion of the pn junction. Therefore, almost no current is flown through the pn junction.

Accordingly, it can be seen that the pn junction acts similarly to the existing pn junction using silicon or the like.

It should be noted that the mechanism of the movement of charges (electrons or electron holes) in a molecule of the protein semiconductor is described in Non-Patent Document 6 and Patent Document 2. According to this, electrons transit between the molecular orbitals when the protein semiconductor is light-excited. As a result, electrons or electron holes are moved from a site of the protein semiconductor to another site.

A specific example of the pn junction will be described.

As the p-type protein semiconductor 21, for example, the p-type zinc-substituted cytochrome c is used, and as the n-type protein semiconductor 22, for example, the n-type zinc-substituted cytochrome b₅₆₂ is used.

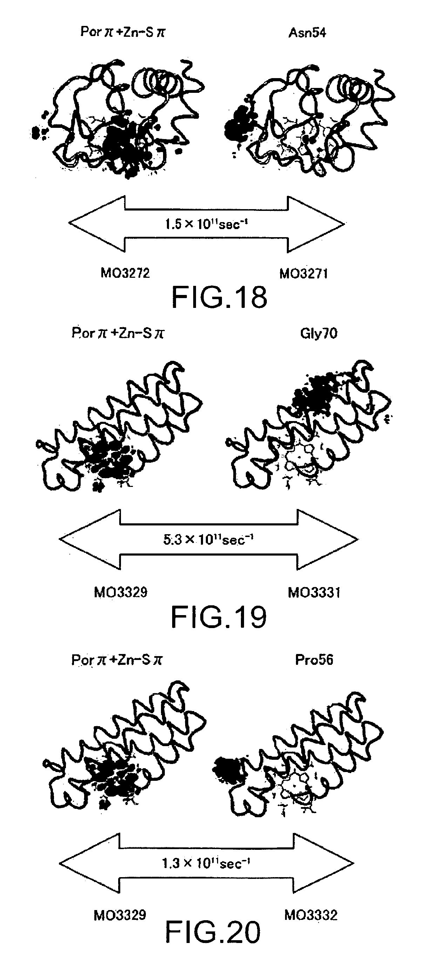

A gateway of a p-channel in the p-type zinc-substituted cytochrome c is a porphyrin ring (Porπ+Zn-Sπ) and Lys7 (Fig. 17), or a porphyrin ring (Porπ+Zn-Sπ) and Asn54 (Fig. 18). The orbital numbers of the molecular orbitals of the porphyrin ring (Porn+Zn-Sn) and Lys7, which are shown in Fig. 17, are 3268 and 3270, respectively, the transition rate of electron holes between the porphyrin ring (Porπ+Zn-Sπ) and Lys7 is 2.0×10¹⁰ sec⁻¹, and the distance between them is 16.5 Å. The orbital numbers of the molecular orbitals of the porphyrin ring (Porn+Zn-Sn) and Asn54, which are shown in Fig. 18, are 3272 and 3271, respectively, the transition rate of electron holes between the porphyrin ring (Porπ+Zn-Sπ) and Asn54 is 1.5×10¹¹ sec⁻¹, and the distance between them is 17.2 Å.

A gateway of an n-channel in the p-type zinc-substituted cytochrome b₅₆₂ is a porphyrin ring (Porπ+Zn-Sπ) and Gly70 (Fig. 19), or the porphyrin ring (Porπ+Zn-Sπ) and Pro56 (Fig. 20). The orbital numbers of the molecular orbitals of the porphyrin ring (Porπ+Zn-Sπ) and Gly70, which are shown in Fig. 19, are 3329 and 3331, respectively, the transition rate of electrons between the porphyrin ring (Porπ+Zn-Sπ) and Gly70 is 5.3×10¹¹ sec⁻¹, and the distance between them is 16.1 Å. The orbital numbers of the molecular orbitals of the porphyrin ring (Porπ+Zn-Sπ) and Pro56, which are shown in Fig. 20, are 3329 and 3332, respectively, the transition rate of electrons between the porphyrin ring (Porn+Zn-Sn) and Pro56 is 1.3×10¹¹ sec⁻¹, and the distance between them is 21.3 Å.

According to the third embodiment, it is possible to attain the pn junction in which the p-type protein semiconductor 21 and the n-type protein semiconductor 22 are joined together. The pn junction has not only similar advantages to those of the existing pn junction but also the following advantages. Specifically, the pn junction can be configured to have an extremely fine structure, i.e., have a size of 4 to 20 nm, because the sizes of the p-type protein semiconductor 21 and the n-type protein semiconductor 22 are 2 to 10 nm. Therefore, in the case where the pn junction is integrated, it is possible to significantly increase the integration density. In the pn junction, because the joining portion has no space charge region unlike the well-known existing pn junction using silicon or the like, the time period when electrons and electron holes go across the joining portion is very short. Therefore, the response rate is extremely high. Moreover, because the sizes of the p-type protein semiconductor 21 and the n-type protein semiconductor 22 are significantly small, i.e., 2 to 10 nm, no problem of being affected by an impurity is caused unlike the well-known existing pn junction using silicon or the like. Therefore, it is possible to increase the quantum efficiency at the time when the pn junction is operated in a forward direction bias mode.

### <4. Fourth Embodiment>

### [Light Emission Element]

In a fourth embodiment, a light emission element using the pn junction according to the third embodiment will be described.

As shown in Fig. 14A, the light emission element is configured by the pn junction in which the p-type protein semiconductor 21 and the n-type protein semiconductor 22 are joined together.

### [Operation of Light Emission Element]

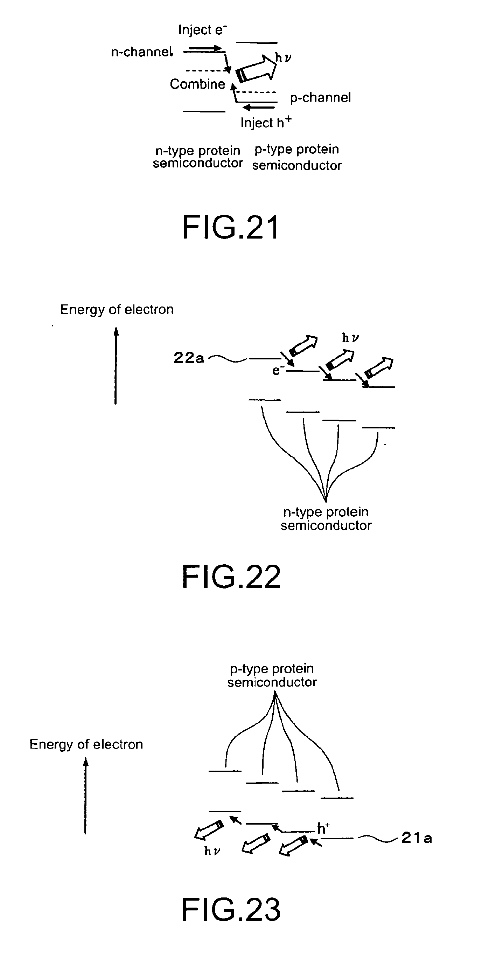

When the light emission element is operated, current is flown through the pn junction in the forward direction by applying a forward bias to the pn junction, specifically, applying voltage between the p-type protein semiconductor 21 and the n-type protein semiconductor 22 so that the potential of the p-type protein semiconductor 21 is higher than that of the n-type protein semiconductor 22. At this time, as shown in Fig. 21, an electron (e⁻) and an electron hole (h⁺) are injected into the joining portion of the pn junction from the p-type protein semiconductor 21 and from the n-type protein semiconductor 22, respectively, and the electron and electron hole are recombined, thereby generating photons (hν). Thus, light is taken out from the light emission element.

In the light emission element, the energy difference between the p-channel 21a and the channel 22a is determined by the voltage applied to the pn junction. Therefore, by controlling the voltage applied to the pn junction, it is possible to control the energy difference between the p-channel 21a and the channel 22a and therefore the wavelength of the light taken out from the light emission element. In other words, the light emission wavelength of the light emission element varies depending on the voltage applied to the pn jucntion. Moreover, in the light emission element, because the electron (e⁻) injected from the p-type protein semiconductor 21 and the electron hole (h⁺) injected from the n-type protein semiconductor 22 are efficiently recombined in the joining portion of the pn junction, it is possible to obtain a light emission element with high efficiency.

According to the fourth embodiment, it is possible to achieve not only the similar advantages to those of the third embodiment but also advantages of obtaining a light emission element with high efficiency and a variable wavelength.

### <5. Fifth Embodiment>

### [Quantum Cascade Laser]

In a fifth embodiment, a quantum cascade laser using the n-type protein semiconductor or the p-type protein semiconductor will be described.

As described above, by controlling the surface charge of the p-type protein semiconductor 21 and the n-type protein semiconductor 22, it is possible to control the energy of the p-channel 21a and the n-channel 22a.

In view of the above, for example, a plurality of types of n-type protein semiconductors 22 are manufactured so that the energy of the n-channel 22a of the n-type protein semiconductor 22 is gradually decreased, and the plurality of types of n-type protein semiconductors 22 are sequentially joined together so that the energy of the n-channel 22a is gradually decreased. Fig. 22 shows the n-type quantum cascade laser thus obtained. Alternatively, a plurality of types of p-type protein semiconductors 21 are manufactured so that the energy of the p-channel 21a of the p-type protein semiconductor 21 is gradually decreased, and the plurality of types of p-type protein semiconductors 21 are sequentially joined together so that the energy of the p-channel 21a is gradually decreased. Fig. 23 shows the p-type quantum cascade laser thus obtained.

As shown in Fig. 22, in the n-type quantum cascade laser, voltage is applied between the n-type protein semiconductor 22 at one end and the n-type protein semiconductor 22 the other end so that the potential of the n-type protein semiconductor 22 in which the energy of the n-channel 22a is the highest is lower than that of the n-type protein semiconductor 22 in which the energy of the n-channel 22a is the lowest. At this time, electrons transit from the n-channel 22a of the n-type protein semiconductor 22 in which the energy of the n-channel 22a is the highest to the n-channel 22a of the n-type protein semiconductor 22 in which the energy of the n-channel 22a is the second highest, and photons (hν) of the energy corresponding to the energy difference between these n-channels 22a at the joining portion of these n-type protein semiconductors 22 are generated. Similarly, electrons transit between the n-channels 22a of a pair of adjacent n-type protein semiconductors 22, and photons of the energy corresponding to the energy difference between them are generated. If the energy differences between the n-channels 22a of pairs of adjacent n-type protein semiconductors 22 are different from each other, it is possible to differentiate wavelengths of light generated from each joining portion from each other. Therefore, according to the n-type quantum cascade laser, it is possible to take out a plurality of light beams having different light emission wavelengths and to obtain the n-type quantum cascade laser having a variable wavelength by selecting the light emission wavelength to be taken out.

Similarly, as shown in Fig. 23, in the p-type quantum cascade laser, voltage is applied between the p-type protein semiconductor 21 at one end and the p-type protein semiconductor 21 at the other end so that the potential of the p-type protein semiconductor 21 in which the energy of the p-channel 21a is the lowest is lower than that of the p-type protein semiconductor 21 in which the energy of the p-channel 21a is the highest. At this time, electron holes transit from the p-channel 21a of the p-type protein semiconductor 21 in which the energy of the p-channel 21a is the lowest to the p-channel 21a of the p-type protein semiconductor 21 in which the energy of the p-channel 21a is the second lowest, and photons (hν) of the energy corresponding to the energy difference between these p-channels 21a at the joining portion of these p-type protein semiconductors 21 are generated. Similarly, electrons transit between the p-channels 21a of a pair of adjacent p-type protein semiconductors 21, and photons of the energy corresponding to the energy difference between them are generated. If the energy differences between the p-channels 21a of pairs of adjacent p-type protein semiconductors 21 are different from each other, it is possible to differentiate wavelengths of light generated from each joining portion from each other. Therefore, according to the p-type quantum cascade laser, it is possible to take out a plurality of light beams having different light emission wavelengths and to obtain the p-type quantum cascade laser having a variable wavelength by selecting the light emission wavelength to be taken out.

According to the fifth embodiment, it is possible to achieve not only the similar advantages to those of the third embodiment but also advantages of obtaining the n-type or p-type quantum cascade laser with high efficiency and a variable wavelength.

### <6. Sixth Embodiment>

### [Bulk-Heterojunction Type Photoelectric Conversion Element]

In a sixth embodiment, a bulk-heterojunction type photoelectric conversion element will be described.

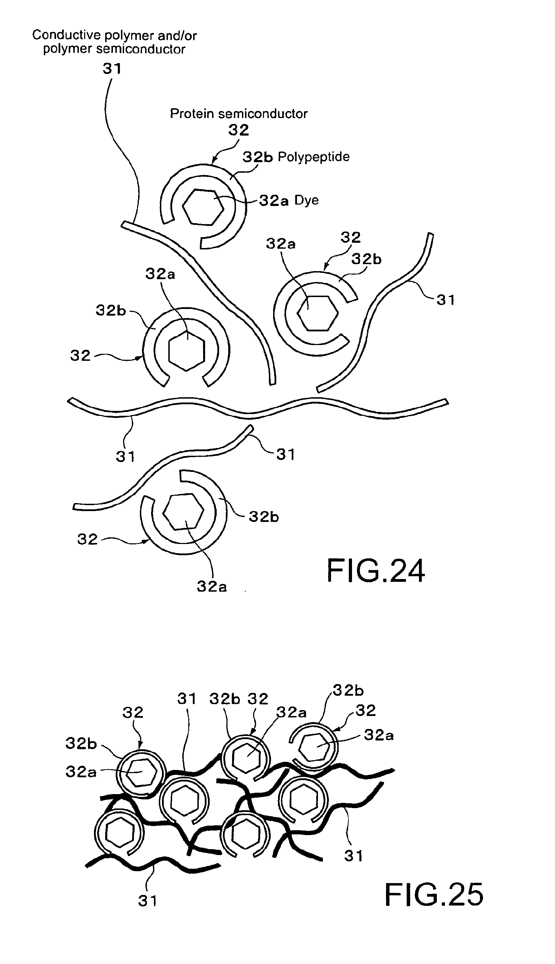

Fig. 24 shows the bulk-heterojunction type photoelectric conversion element.

As shown in Fig. 24, the bulk-heterojunction type photoelectric conversion element has a structure in which a heterojunction is formed by a network-like conductive polymer and/or polymer semiconductor 31 complicated with one or more of p-type or n-type protein semiconductors 32, for example. The protein semiconductor 32 has a long-lived excited state, and is obtained by aligning a dye 32a being a light emission center, which is covered in a polypeptide 32b, at a predetermined position. Here, the "long-lived" in the protein semiconductor 32 having a long-lived excited state means a lifetime of the excited state, which is common to a fluorescent dye or phosphorescent dye, and is typically, but not limited to, several ten pico-seconds or more. Typically, the conductive polymer and/or polymer semiconductor 31 and the protein semiconductor 32 are bonded by a non-covalent bond or a covalent bond. Examples of the non-covalent bond include an electrostatic interaction, a van der Waals interaction, a hydrogen bonding interaction, and a charge transfer interaction. The conductive polymer and/or polymer semiconductor 31 and the protein semiconductor 32 may be bonded by a linker (not shown).

The conductive polymer and/or polymer semiconductor 31 may be p-type or n-type. There are two main types of the conductive polymer: a hydrocarbon-based conductive polymer and a hetero atom-containing conductive polymer. Examples of the hydrocarbon-based conductive polymer include polyacetylene, polyphenylene, polyphenylene vinylene, polyacene, polyphenyl acetylene, polydiacetylene, and polynaphthalene. Examples of the hetero atom-containing conductive polymer include polypyrrole, polyaniline, polythiophene, polythienylene vinylene, polyazulene, and polyisothianaphthene.

The bulk-heterojunction type photoelectric conversion element is formed on a substrate as necessary, in order to mechanically support the bulk-heterojunction type photoelectric conversion element, for example. As the substrate, a well-known existing substrate can be used, is selected as necessary, and may be a transparent substrate or a non-transparent substrate. The material of the transparent substrate is selected as necessary. Examples of the material include transparent inorganic materials such as quartz and glass, and transparent plastic. As a flexible transparent substrate, a transparent plastic substrate is used. Examples of the transparent plastic include polyethylene terephthalate, polyethylene naphthalate, polycarbonate, polystyrene, polyethylene, polypropylene, polyphenylene sulphide, polyvinylidene difluoride, acetylcellulose, brominated phenoxy, aramids, polyimides, polystyrenes, polyarylates, polysulfones, and polyolefins. As the transparent substrate, a silicon substrate is used, for example.

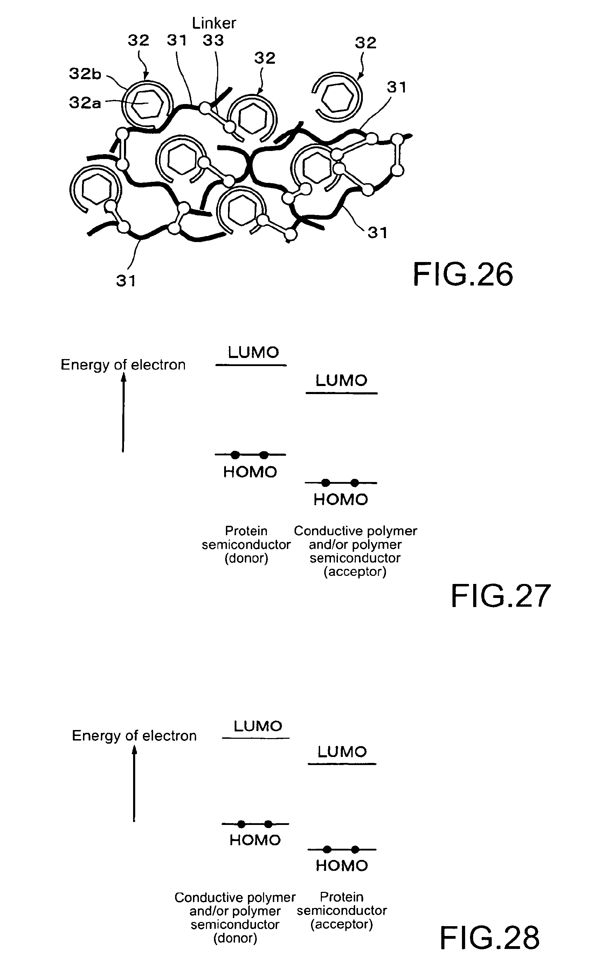

Fig. 25 schematically shows an exemplary state where the conductive polymer and/or polymer semiconductor 31 and the protein semiconductor 32 are bonded by a non-covalent bond. Moreover, Fig. 26 schematically shows an exemplary state where the conductive polymer and/or polymer semiconductor 31 and the protein semiconductor 32 are bonded by a linker 33.

As the linker 33, a well-known existing linker can be used, and is selected depending on the conductive polymer and/or polymer semiconductor 31 and the protein semiconductor 32 as appropriate.

Fig. 27 shows an exemplary energy band of the bulk-heterojunction type photoelectric conversion element. As shown in Fig. 27, in the bulk-heterojunction type photoelectric conversion element, the HOMO (highest occupied molecular orbital) and LUMO (lowest unoccupied molecular orbital) of the protein semiconductor 32 are higher than the HOMO and the LUMO of the conductive polymer and/or polymer semiconductor 31. In this case, the protein semiconductor 32 is n-type. The conductive polymer and/or polymer semiconductor 31 acts as an acceptor, and the protein semiconductor 32 acts as a donor. In the bulk-heterojunction type photoelectric conversion element, if the n-type protein semiconductor 32 being a donor absorbs light incident from the outside, electrons (represented by black dots in Fig. 27) are excited from the HOMO into the LUMO in the protein semiconductor 32, and excitons are formed. The electrons are moved to the LUMO of the p-type conductive polymer and/or polymer semiconductor 31 being an acceptor. As a result, a charge separation state in which the protein semiconductor 32 has positive charges (electron holes) and the conductive polymer and/or polymer semiconductor 31 has negative charges (electrons) is generated. After the charge separation state is generated in this way, electron holes move in the protein semiconductor 32, and electrons move in the conductive polymer and/or polymer semiconductor 31. The electron holes and electrons are taken out to the outside, and photocurrent is obtained.

Fig. 28 shows another exemplary energy band of the bulk-heterojunction type photoelectric conversion element. As shown in Fig. 28, in the bulk-heterojunction type photoelectric conversion element, the HOMO and the LUMO of the conductive polymer and/or polymer semiconductor 31 are higher than the HOMO and LUMO of the protein semiconductor 32. In this case, the protein semiconductor 32 is p-type. The conductive polymer and/or polymer semiconductor 31 acts as a donor, and the protein semiconductor 32 acts as an acceptor. In the bulk-heterojunction type photoelectric conversion element, if the conductive polymer and/or polymer semiconductor 31 being a donor absorbs light incident from the outside, electrons are excited from the HOMO into the LUMO in the conductive polymer and/or polymer semiconductor 31, and excitons are formed. The electrons are moved to the LUMO of the p-type protein semiconductor 32 being an acceptor. As a result, a charge separation state in which the conductive polymer and/or polymer semiconductor 31 has positive charges (electron holes) and the protein semiconductor 32 has negative charges (electrons) is generated. After the charge separation state is generated in this way, electron holes move in the HOMO of the conductive polymer and/or polymer semiconductor 31, and electrons move in the protein semiconductor 32. The electron holes and electrons are taken out to the outside, and photocurrent is obtained.

Examples of the p-type conductive polymer and/or polymer semiconductor 31 include p-type polyaniline sulfonic acid (PASA)

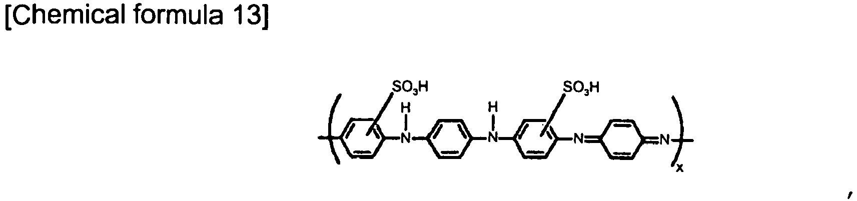

### poly[2-methoxy-5-(2'-ethyl-hexyloxy)-1,4-phenylene vinylene] (MEH-PPV)

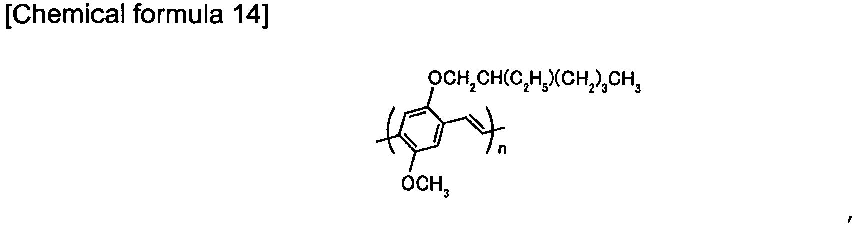

and

### poly(3-hexylthiophene) (P3HT)

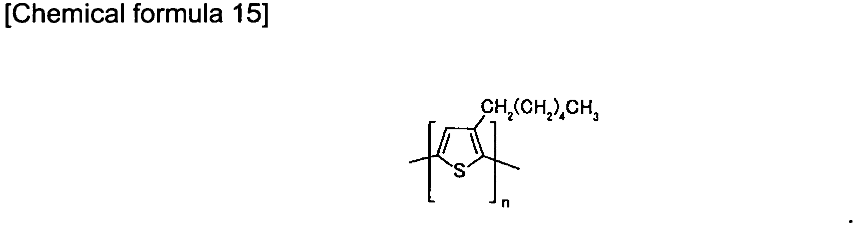

As the n-type conductive polymer and/or polymer semiconductor 31, Poly(p-pyridyl vinylene)Poly(isothianaphthene) can be used, for example.

A specific example of the bulk-heterojunction type photoelectric conversion element will be described.

As the p-type conductive polymer and/or polymer semiconductor 31, the p-type polyaniline sulfonic acid (PASA) is used. As the protein semiconductor 32, the zinc-substituted cytochrome c is used.

A protein semiconductor solution is prepared by dissolving the zinc-substituted cytochrome c in water. Moreover, a polyaniline sulfonic acid (PASA) solution is prepared by dissolving PASA in water. A protein semiconductor polymer aqueous solution is prepared by adding the PASA solution thus prepared to the above-mentioned protein semiconductor solution.

By neutralizing the sulfonic acid group of the PASA in the protein semiconductor polymer aqueous solution with alkali, e.g., sodium hydroxide (NaOH), it is possible to control the pH of the protein semiconductor polymer aqueous solution. By selecting the optimal ratio of the alkali and the sulfonic acid group, it is possible to control the band position (energy of LUMO and HOMO) of the zinc-substituted cytochrome c so that the quantum efficiency of the bulk-heterojunction type photoelectric conversion element is maximized.

According to the sixth embodiment, it is possible to achieve not only the similar advantages to those of the third embodiment but also advantages of obtaining a bulk-heterojunction type photoelectric conversion element with high efficiency. The bulk-heterojunction type photoelectric conversion element can be used as a light-receiving element (photo sensor), a solar cell, or the like.

### <7. Seventh Embodiment>

### [Electric Field Detection Element]

In a seventh embodiment, an electric field detection element will be described.

The electric field detection element includes the p-type protein semiconductor, the n-type protein semiconductor, or the pn junction obtained by joining the p-type protein semiconductor and the n-type protein semiconductor.

The operation of the electric field detection element will be described.

If the Hamiltonian of the electric field detection element in the electric field is represented by H, the relationship, H=H₀ +H₁, is established. Here, H₀ represents the zero-order Hamiltonian, and H₁ represents the first-order Hamiltonian (first-order perturbation). H₁ is a value obtained by multiplying the dipole moment in a z direction by the electric field ε, and the relationship, H₁ =ezε, is established. Here, e represents an electron charge.

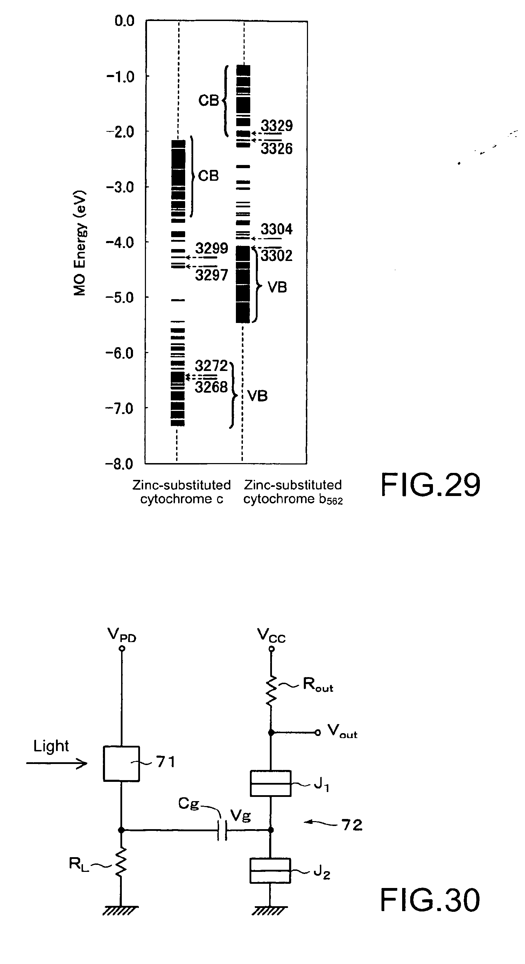

Fig. 29 shows the energy of the molecular orbitals of the zinc-substituted cytochrome c and the zinc-substituted cytochrome b₅₆₂. The VB represents the valance band, and the CB represents the conduction band. Numbers on the side of the molecular orbitals represent molecular orbital number. In the zinc-substituted cytochrome c, four molecular orbitals 3268, 3272, 3297, and 3299 are pi-orbitals or pi-star orbitals of porphyrin, and other molecular orbitals are those of amino acid residues. Similarly, in the zinc-substituted cytochrome b₅₆₂, four molecular orbitals 3302, 3304, 3326, and 3329 are pi-orbitals or pi-star orbitals of porphyrin, and other molecular orbitals are those of amino acid residues. Because these four molecular orbitals have the directionality, the effect of the electric field significantly varies depending on the direction of the electric field applied. On the other hand, because other molecular orbitals are isotropic, the effect of the electric field is equally generated. Therefore, the band sift of the amino acid residues is an averaged one. On the other hand, the four molecular orbitals are significantly shifted if the electric field is applied from the z direction, i.e., the pz orbital is turned into the pi-orbital. On the other hand, the four molecular orbitals are little affected by the electric field applied from the x-direction or the y-direction.

As described above, the relationship between the band of the amino acid residues shown in Fig. 29 and the above-mentioned four molecular orbitals significantly varies depending on the electric field applied. For example, those acting as the n-type protein semiconductor if the electric field is applied from the z-direction act as the p-type protein semiconductor or show almost no photocurrent if the electric field is applied from the x-direction or the y-direction. If the intensity of the electric field is, for example, 1 MV/m, it is considered that the band shift of, for example, about 0.01 eV to 0.1 eV, can be observed.

As described above, according to the seventh embodiment, it is possible to attain a novel electric field detection element. According to the electric field detection element, by arranging the electric field detection element at the site for detecting the electric field to be measured, it is possible to detect the electric field by using the above phenomenon. Because the electric field detection element can be configured to have an extremely fine structure, i.e., have a size of several nm to several ten nm, it is possible to measure the electric field in the extremely small area having a size of nm order, which is difficult to measure in the past, or to measure the distribution of the electric field with high accuracy. The electric field detection element is suitable for use to measure the strong electric field particularly.

### <8. Eighth Embodiment>

### [Bipolar Transistor]

In an eighth embodiment, a bipolar transistor will be described.

By sequentially joining the p-type protein semiconductor, the n-type protein semiconductor, and the p-type protein semiconductor together, it is possible to configure a pnp-type bipolar transistor. Alternatively, by sequentially joining the n-type protein semiconductor, the p-type protein semiconductor, and the n-type protein semiconductor together, it is possible to configure an npn-type bipolar transistor.

According to the eights embodiment, it is possible to attain a novel bipolar transistor. The bipolar transistor can be used for various usages, and can be used as, for example, a photo transistor.

### <9. Ninth Embodiment>

### [Thyristor]

In a ninth embodiment, a thyristor will be described.

The thyristor is a pnpn-type thyristor configured by sequentially joining the p-type protein semiconductor, the n-type protein semiconductor, the p-type protein semiconductor, and the n-type protein semiconductor together.

According to the ninth embodiment, it is possible to attain a novel thyristor. The thyristor can be used for various usages.

### <10. Tenth Embodiment>

### [Photo Sensor]

Fig. 30 is a circuit diagram showing a photo sensor according to a tenth embodiment.

As shown in Fig. 30, the photo sensor is configured of a photodiode 71 including the bulk-heterojunction type photoelectric conversion element according to the sixth embodiment, and a single electron transistor 72 for amplifying the output of the photodiode 71. The single electron transistor 72 includes a micro tunnel junction J₁ on the drain side, and a micro tunnel junction J₂ on the source side. The capacities of these micro tunnel junctions J₁ and J₂ are referred to as C₁ and C₂, respectively. For example, one electrode of the photodiode 71 is grounded via a load resistance R_{L}, and the other electrode is connected to a positive power supply for supplying a positive voltage V_{PD} for biasing the photodiode 72. On the other hand, the source of the single electron transistor 72 is grounded, and the drain thereof is connected to a positive power supply that supplies a positive voltage V_{cc} via an output resistance Rout. Then, the electrode on the side of the load resistance R_{L} of the photodiode 71 and a gate of the single electron transistor 72 are connected to each other via a capacity Cg.

In the photo sensor configured as described above, the capacity Cg is charged by voltage generated at both ends of the load resistance R_{L} when light is applied to the photodiode 71 and photocurrent is flown. A gate voltage Vg is applied to the gate of the single electron transistor 72 via the capacity Cg. Then, by measuring a change ΔQ=Cg ΔVg in the amount of charge accumulated in the capacity Cg, a change ΔVg in the gate voltage Vg is measured. Here, the single electron transistor 72 used for amplifying the output of the photodiode 71 can measure a change ΔQ=Cg ΔVg in the amount of charge accumulated in the capacity Cg with the sensitivity 1 million higher than that of the existing transistor, for example. Specifically, because the single electron transistor 72 can measure a change ΔVg in the minute gate voltage Vg, it is possible to reduce the value of the load resistance R_{L}. Accordingly, it is possible to significantly increase the sensitivity and speed of the photo sensor. Moreover, because thermal noise is suppressed by the charging effect on the side of the single electron transistor 72, it is possible to suppress the noise generated on the side of the amplifying circuit. Furthermore, because the single electron transistor 72 uses a tunneling effect of one electron in its basic operation, the power consumption is extremely low.

As described above, in the photo sensor, the photodiode 71 and the single electron transistor 72 are capacitively-coupled. Because the voltage gain at this time is given by Cg /C₁, by sufficiently reduce the capacity C₁ of the micro tunnel junctionJ₁, it is possible to easily obtain the output voltage Vₒᵤₜ large enough to drive the element connected to the next stage of the photo sensor.

As described above, according to the tenth embodiment, it is possible to attain a novel photo sensor using a protein semiconductor, which can be reliably used for a long time. Moreover, the photo sensor is configured so that the single electron transistor 72 amplifies the output of the photodiode 71. Therefore, it is possible to significantly increase the speed and sensitivity of the photo sensor, and to reduce the power consumption, as compared to the existing general photo sensor that amplifies the output of the photodiode by the existing general transistor.

### <11. Eleventh Embodiment>

### [Inverter Circuit]

Next, an inverter circuit according to an eleventh embodiment will be described.

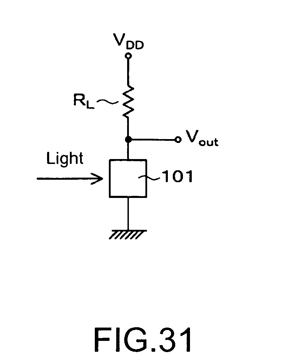

Fig. 31 shows the inverter circuit. As shown in Fig. 31, in the inverter circuit, a photoelectric conversion element 101 having the similar configuration to that of the bulk-heterojunction type photoelectric conversion element according to the sixth embodiment and the load resistance R_{L} are connected in series. A predetermined positive power supply voltage V_{DD} is applied to one end of the load resistance R_{L}, and the electrode is grounded. If light at the absorption wavelength of a photoelectric conversion element 101 as signal light is applied to the photoelectric conversion element 101, the photoelectric conversion element 101 is turned on, and photocurrent is flown. Accordingly, the output voltage Vₒᵤₜ from an electrode (not shown) becomes low level. If the irradiation of light is stopped, the photoelectric conversion element 101 is turned off, and photocurrent is caused not to flow. Accordingly, the output voltage Vₒᵤₜ from the electrode becomes high level.

According to the ninth embodiment, it is possible to configure a novel inverter circuit using a protein semiconductor, which can be reliably used for a long time, and to configure various circuits such as logical circuits by using the inverter circuit.

Although embodiments and examples have been specifically described, the present disclosure is not limited to the above-mentioned embodiments and examples, and various modifications can be made based on technical ideas of the present technology.

For example, the numerical value, structure, configuration, shape, material, and the like described in the above-mentioned embodiments and examples are only examples,
and different numerical value, structure, configuration, shape, material, and the like may be used as necessary.

### Description of Reference Symbols

- 11: gold electrode
- 13: self-assembled monolayer
- 21: p-type protein semiconductor
- 21a: p-channel
- 22: n-type protein semiconductor
- 22a: n-channel
- 31: conductive polymer and/or polymer semiconductor
- 32: protein semiconductor

## Claims

1. A method of manufacturing a protein semiconductor, comprising
controlling a conductivity type of the protein semiconductor by controlling total amount of charge in amino acid residues.

2. The method of manufacturing a protein semiconductor according to claim 1, wherein
the total amount of charge in amino acid residues is controlled by
substituting one or more of an acidic amino acid residue, a basic amino acid residue, and a neutral amino acid residue, which are contained in protein, with an amino acid residue having different properties,
adding one or more of an acidic amino acid residue, a basic amino acid residue, and a neutral amino acid residue to the protein,
deleting one or more of an acidic amino acid residue,
a basic amino acid residue, and a neutral amino acid residue, which are contained in the protein,
chemically modifying one or more of an acidic amino acid residue, a basic amino acid residue, and a neutral amino acid residue, which are contained in the protein, or
controlling polarity of a medium surrounding the protein.

3. The method of manufacturing a protein semiconductor according to claim 2, wherein
the protein is electron transfer protein.

4. The method of manufacturing a protein semiconductor according to claim 3, wherein
the electron transfer protein contains metal.

5. The method of manufacturing a protein semiconductor according to claim 4, wherein
the electron transfer protein is zinc-substituted cytochrome c or zinc-substituted cytochrome b₅₆₂.

6. A protein semiconductor whose conductivity type is controlled by controlling total amount of charge in amino acid residues.

7. A method of manufacturing a pn junction, comprising:
manufacturing a p-type protein semiconductor and an n-type protein semiconductor by controlling total amount of charge in amino acid residues; and
manufacturing a pn junction by joining the p-type protein semiconductor and the n-type protein semiconductor together.

8. A pn junction manufactured by
manufacturing a p-type protein semiconductor and an n-type protein semiconductor by controlling total amount of charge in amino acid residues, and
joining the p-type protein semiconductor and the n-type protein semiconductor together.

9. A method of manufacturing a semiconductor apparatus, comprising the steps of:
manufacturing a p-type protein semiconductor and an n-type protein semiconductor by controlling total amount of charge in amino acid residues; and
manufacturing a pn junction by joining the p-type protein semiconductor and the n-type protein semiconductor together.

10. The method of manufacturing a semiconductor apparatus according to claim 9, wherein
the semiconductor apparatus is a light-receiving element or a light emission element.

11. A semiconductor apparatus, comprising
a pn junction manufactured by
manufacturing a p-type protein semiconductor and an n-type protein semiconductor by controlling total amount of charge in amino acid residues, and
joining the p-type protein semiconductor and the n-type protein semiconductor together.

12. An electronic apparatus, comprising
a semiconductor apparatus including
a pn junction manufactured by
manufacturing a p-type protein semiconductor and an n-type protein semiconductor by controlling total amount of charge in amino acid residues, and
joining the p-type protein semiconductor and the n-type protein semiconductor together.

13. A method of controlling a conductivity type of a protein semiconductor, comprising
controlling the conductivity type of the protein semiconductor by controlling total amount of charge in amino acid residues.
